# EUROPEAN PATENT APPLICATION

(11) **EP 1 947 583 A1**
(43) Date of publication of application: **23.07.2008**
(21) Application number: 07000871.9
(22) Date of filing: 17.01.2007
(51) Int. Cl.: G06F 19/00, G06F 15/02

(54) **Device for controlling the caloric contents of alimentary diets**

(71) Applicant: Da Villa, Giuliano, 80121 Napoli (IT)
(72) Inventor: Da Villa, Giuliano, 80121 Napoli (IT)
(74) Representative: Cicogna, Franco

(57) **Abstract**

The present invention relates to a device for controlling the caloric contents of alimentary diets, comprising a central unit, coupled to a display and controlled by a digital keyboard and a push-button for selecting food products or combinations of food products as stored in a fixed memory.

Said fixed memory further stores therein biologic data of a user for calculating the user caloric requirements, and data related to a unit value of food products or combinations of food products, as calculated per gram of nourishing substances or food products.

The central unit processes the caloric value of each food product to be assumed, depending on its amount and evaluates the caloric compatibility thereof with the energetic requirement of the user.

## Description

### BACKGROUND OF THE INVENTION

The present invention relates to a device for controlling the caloric contents of alimentary diets.

As is known, the control of the caloric contents of food products to be assumed and/or administered for performing a proper alimentary diet is very important in a number of disease cases, such as, for example, obesity, renal insufficiency, hepatopathies, hypercholesteremia, hypertrigliceridemia and dislipidemic diseases in general, as well as in gout, stomach and digestive tract diseases and the like.

In these events, it is indispensable to provide a proper knowledge of the caloric value of the assumed food products and their nourishing substance compositions such as glucides, lipides and proteins.

A calculation of the total caloric amount, related to one or more full meals would involve a lot of rather complex operations, to detect the unit caloric value of the individual food products, which value must be multiplied by the weight of the food products, and must be saved for performing addition operations and the like.

Thus, a proper control of the caloric amount, so performed as to provide precise and accurate results, by a user, is a rather difficult operation.

### SUMMARY OF THE INVENTION

Accordingly, the aim of the present invention is to solve the above mentioned problems, by providing a device for controlling the caloric contents of alimentary diets, which allows to perform an immediate calculation of the caloric contents of a given amount of food products, thereby allowing to immediately provide an evaluation of the compatibility of said food products with a single person or user.

Within the scope of the above mentioned aim, a main object of the invention is to provide such a calculating device adapted to provide a broad range of information, in a very simple manner, to allow the user to customize the device or instrument based on his/her personal diet requirements.

Yet another object of the present invention is to provide such an alimentary diet caloric contents calculation device which, owing to its specifically designed construction and mode of operation, is very reliable and safe in operation.

Yet another object of the present invention is to provide such a device or instrument which can be easily made and which, moreover, is very competitive from a mere economic standpoint.

According to one aspect of the present invention, the above mentioned aim and objects, as well as yet other objects, which will become more apparent hereinafter, are achieved by a device for controlling the caloric contents of alimentary diets, characterized in that said device comprises a central processing unit coupled to a display and controlled by a first digital keyboard and a selection pushbutton for displaying on said display food products to be consumed and the related caloric contents thereof.

Said device comprises moreover a fixed memory, in which can be stored biologic data of a user for calculating the user caloric requirements of the latter, as well as data related to a unit caloric contents of said food products or combinations thereof, as calculated per gram of nourishing substances.

Thus, it is possible to evaluate, on a meal by meal basis, the caloric compatibility of the food products to be assumed, and related energetic power for each user.

Moreover, by said fixed memory, it is possible to provide the user with an information of the caloric contents assumed in a single meal, in several daily meals and/or weekly meals.

Said central unit is designed for processing the caloric value or contents of the food products to be assumed in relationship with their amount and/or caloric compatibility with the user energetic requirements.

### BRIEF DESCRIPTION OF THE DRAWING

Further characteristics and advantages of the present invention will become more apparent hereinafter from the following detailed disclosure of a preferred, though not exclusive, embodiment of a device for controlling the caloric contents of alimentary diets, which is illustrated, by way of an indicative, but not limitative, example in the accompanying drawing, where:

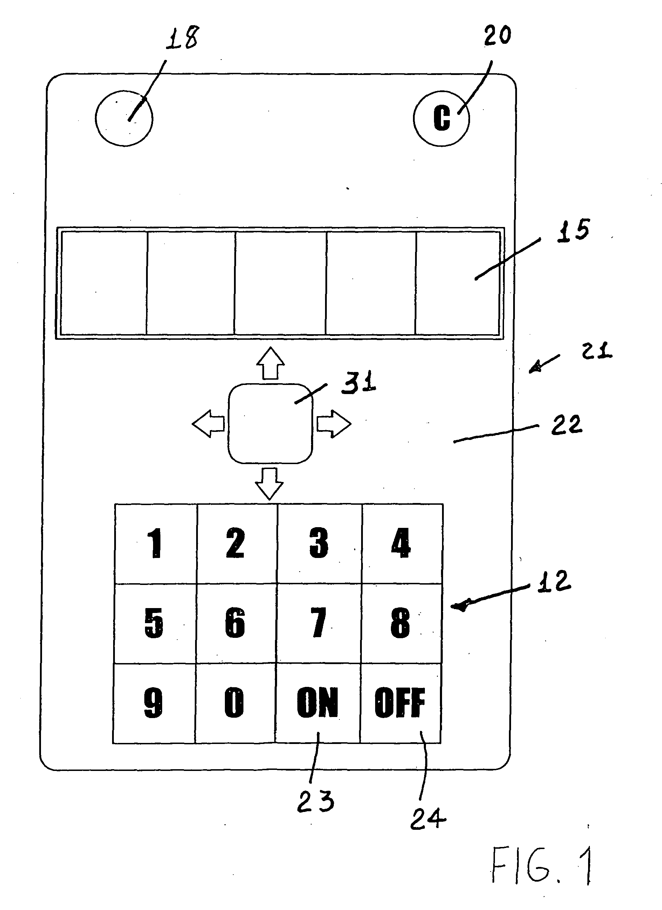

Figure 1 is a schematic top plan view of the device according to the invention.

### DESCRIPTION OF THE PREFERRED EMBODIMENT

With reference to the number references of the above mentioned figure, the device for controlling the caloric contents of alimentary diets, according to the present invention, which has been generally indicated by the reference number 21, comprises a central processing unit, arranged in a box-like body or housing 22.

Said box-like body has a cross top opening housing a display therein, said display being preferably of an LCD or liquid crystal type.

The subject control device comprises moreover two push-button elements.

In particular, of said push-button elements, the push-button 20 is used for clearing data displayed on the display, whereas the other push-button, indicated by the reference letters OK, is used for confirming a setting of a given or target operation.

At the bottom of said display 15 a further swinging selecting push-button element 31, for selecting stored data which are stored in memory means of the device 1, is moreover provided.

Said swinging selecting push-button 31 can be up and down, as well as rightward and leftward displaced.

A further plurality of push-buttons or keys, generally indicated by the reference numbers 1, 2, 3, 4, 5, 6, 7, 8, 9 and 0 are used for setting a lot of digital values to be stored in the memory means of the device 21 according to the invention, together with several parameters and means distinguishing food products to be displayed on the display.

Finally, two further keys 23 and 24, indicated by OFF and ON, for switching off and on the subject device are also provided.

As stated, the subject device comprise memory means, controlled by a controlling software, allowing to properly program the device by entering proper biologic data related to the user, such as, for example, his/her age, sex, height, weight and physical exercising activity.

Thus, the device 21, as programmed in the thereinabove disclosed manner, will be able of accurately establishing the energetic requirements of the user.

To that end, by operating the selection push-button 31, it is possible to display and successively scroll on said display means 15, words indicative of the age, sex, height, weight of the user, and, at said displayed words, the user will digit her/his actual age.

In particular, as the swinging selection push-button 31 is operated, and as the word "sex" appears, the user will press on the key 1 or 2 to input her/his sex, either male or female.

Likewise, as the word "height" appears, then the user will set on the digital keyboard a number value corresponding to his/her height, for example expressed in centimeters.

With respect to the user weight, as the word "weight" appears on the display, then, by operating the selection push-button, the user will set a digital datum corresponding to her/his weight, for example expressed in Kg.

On the other hand, as on the display the words "physical exercising" appear, then the user will program the subject device by operating the keys 1 or 2 or 3 depending on the physical exercises usually performed by the user.

By way of an example, the key 1 is used for small physical exercises, the key 2 for discrete physical exercises and the key 3 for intensive physical exercises.

Thus, the subject device, in addition to calculating the food product caloric contents is also designed for giving the food caloric compatibility to relate the latter with the user energetic requirements and the caloric contents already assumed in a given time period, such as a day, a week, a month, and so on.

In fact, after having programmed his biologic data, comprising, as aforesaid, the age, sex, height, weight and physic exercises, the device will display on its display means the calorie numbers required for a proper feeding.

The displayed information, in each calculation operation, both with respect to the caloric contents of the food products and their energetic compatibility, can be individually detected on the display means during partial calculation operations for calculating the caloric contents of the food products, depending on the specifically set amounts.

Moreover, the display will also display symbols indicating either the compatibility or incompatibility of a preset food products, from the caloric contents standpoint.

By also operating slider keys controlling the stored data, the device will also provide the possibility, after having set the age, sex, height, weight and physical exercising values, of successively displaying, for example in an alphabetic order, the names of main food products which may be used by the user.

Said food products can comprise, for example, pasta, bread, meat, fruits and the like.

Thus, as the display 15 displays a preset food product, in order to calculate the energetic requirements of a given person, the weight in grams of each food product to be assumed is indicated.

Thus, by adding the caloric values of a lot of food product amounts as programmed, it is possible to finally evaluate the amounts of calories, that the food products, assumed in target amounts, will provide to the user.

If the set data exceed the amount of calories required by a user, then the device or apparatus 21 will signal such a caloric incompatibility and, if desired, will also provide a sound alarm.

As stated, the subject device further comprises a clearing key for clearing the partial memory 18.

The thus made device allows to clearly show the caloric contents of the food products to be consumed, starting from their amount by weight, and by also subdividing the calories by grams of nourishing substances, such as proteins, glucides and lipides.

Thus, by displaying on the device display the weight of the single food products constituting a meal, the device allows to easily detect, through the display 15, the caloric power of the individual food products and, if required, of the full meal.

The total caloric value of each meal will be held in the memory means of the device for a presettable time period, thereby providing an useful information related to the caloric contents of several following meals, within a preset dietetic protocol, which can be easily related to a user type or size.

Thus, based on the user biologic data, it will be also possible to indicate the user caloric requirements.

Since the data are saved in the above mentioned memory means, the user can recover therefrom at any time of a day, for example, values or data related to the already assumed calories, thereby following a properly controlled dietetic regimen.

From the above disclosure it should be apparent that the invention fully achieves the intended aim and objects.

In fact, the invention provides a very practical and functional device for controlling the caloric contents of alimentary diets, in general.

The invention, as disclosed, is susceptible to several modifications and variations, all of which will come within the scope of the invention.

In practicing the invention, all the constructional details can be replaced by other technically equivalent elements.

## Claims

1. A device for controlling the caloric contents of alimentary diets, **characterized in that** said device comprises a central processing unit coupled to a display and controlled by a digital keyboard and a selection push-button, said device further comprising a fixed memory for storing biologic data of a user for calculating the caloric requirements of said user, and data related to a unit value of food products or a combination of food products as calculated in grams of nourishing substances, said central processing unit processing the caloric contents of the food products to be assumed depending on their amount and evaluating a caloric compatibility with a user energetic requirement.

2. A device according to the preceding claim, **characterized in that** said device comprises a box-like body including a plurality of windows for housing therein said keyboard and display.

3. A device according to the preceding claims, **characterized in that** said display is a liquid crystal display.

4. A device, according to one or more of the preceding claims, **characterized in that** said device further comprises a clearing key for clearing a partial memory, a further clearing key for clearing the full memory, a switch-on key, a selection push-button and a switch-off key.

5. A device, according to one or more of the preceding claims, **characterized in that** said selecting push-button is a swinging selecting push-button operated for setting a plurality of operating functions of said device.

6. A device, according to one or more of the preceding claims, **characterized in that** said swinging push-button may be upward/downward and rightward/leftward driven.

7. A device according to claim 1, **characterized in that** said memory is controlled by a controlling software allowing said device to be programmed by an input of a user biologic data, comprising the user age, sex, height, weight and physical exercise activity.

8. A device according to claim 1, **characterized in that** said device is so programmed as to establish the user energetic requirements by operating said selection swinging push-button to cause said display to subsequently display words indicating the age, sex, height, weight of said user, whereat said user can key her/his age, sex, height and weight.

9. A device according to claim1, **characterized in that,** as said swinging push-button is operated, said display displays, in succession, the sex of the user which can be programmed by pressing a key depending on the sex of the user.

10. A device, according to one or more of the preceding claims, **characterized in that** said device is so programmed that, as a word "height" is displayed, then the user must set on the keyboard a number value corresponding to his/her height in centimeters.

11. A device, according to one or more of the preceding claims, **characterized in that** said device is so programmed that, as said display displays the word "weight", then the user must set a digital data corresponding to his/her weight in kilograms.

12. A device, according to one or more of the preceding claims, **characterized in that** said device is so programmed that, as said display displays the words "physical exercise activity", then said user must program said device by operating a corresponding programming key depending on the performed physical exercise activity, said keyboard including a key related to a small physic exercise activity, a further key related to a moderate physical exercise activity and a further key related to an intensive physical exercise activity.

13. A device, according to one or more of the preceding claims, **characterized in that** said device is adapted as to evaluate, in addition to calculating the caloric contents of said food products, their caloric compatibility with the energetic requirements of said user and the caloric amount already assumed in a set time, such as a day, a week, a month and so on.

14. A device, according to one or more of the preceding claims, **characterized in that** said device is so designed that, after having programmed the biologic data of the user thereon, comprising the user age, sex, height, weight and performed physical exercise activity, said device displays on said display a number of calories necessary for providing a proper feeding of the user.

15. A device, according to one or more of the preceding claims, **characterized in that** in said device the displaying of each calculating operation, both related to the caloric contents of the food products and to their energetic compatibility, may be detected individually on said display during the calculating operation for partially calculating the caloric contents of said food products, based on their set amount.

16. A device, according to one or more of the preceding claims, **characterized in that** said display is also designed for displaying a plurality of symbols indicating either a caloric compatibility or a caloric incompatibility of a preset food product.

17. A device, according to one or more of the preceding claims, **characterized in that** said device is so programmed that, by operating control means for controlling data stored therein, said device allows after having set age, sex, height, weight and performed physical exercise activity values, to successively display, in an alphabetic order, a name of food products to be used by said user.

18. A device, according to one or more of the preceding claims, **characterized in that** said device, as said display displays thereon a preset food product, for calculating the energetic requirement of a user, provides to key in a weight in grams of each food product to be assumed by the user, and thereby, by adding the caloric contents of the food products amount which have been set, allows to evaluate the caloric amount of said food products in set amounts thereof.

19. A device, according to one or more of the preceding claims, **characterized in that,** if the set data exceeds the calorie amounts necessary for the user, then said device signals such a caloric incompatibility by signaling means, such as a sound signaling means.
